**Europäisches Patentamt**

**European Patent Office**

**Office européen des brevets**

(19)

(11) Veröffentlichungsnummer: **0 146 794**
**B1**

(12) **EUROPÄISCHE PATENTSCHRIFT**

(45) Veröffentlichungstag der Patentschrift:
14.06.89

(51) Int. Cl.⁴: **A 61 F 2/06**

(21) Anmeldenummer: **84114231.8**

(22) Anmeldetag: **24.11.84**

(54) Verfahren zur Herstellung einer Gefässprothese.

(30) Priorität: **16.12.83 DE 3345513**

(43) Veröffentlichungstag der Anmeldung:
**03.07.85 Patentblatt 85/27**

(45) Bekanntmachung des Hinweises auf die Patenterteilung:
**14.06.89 Patentblatt 89/24**

(84) Benannte Vertragsstaaten:
**AT BE CH DE FR GB IT LI LU NL SE**

(56) Entgegenhaltungen:
**DE-C- 1 566 319**
**GB-A- 1 511 030**

(73) Patentinhaber: **B. Braun-SSC AG, Gerliswilstrasse 74,
CH-6020 Emmenbrücke (CH)**

(72) Erfinder: **Braun, Bernd, Dr. Dipl-Chem., Tränkelücke 1,
D-3508 Melsungen (DE)**

(74) Vertreter: **von Kreisler, Alek, Dipl.-Chem. et al,
Deichmannhaus am Hauptbahnhof, D-5000 Köln 1 (DE)**

## Beschreibung

Die Erfindung bezieht sich auf ein Verfahren zur Herstellung einer Gefäßprothese, vornehmlich einer Gefäßprothese mit kleinerem Durchmesser, die zum Ersatz krankhafter oder unbrauchbar gewordener Blutgefäße und Gefäßabschnitte Verwendung finden kann. Gefäßprothesen mit Durchmessern von ca. 5 mm und darunter sind bislang nur mit teilweise zufriedenstellenden mechanischen Eigenschaften herstellbar gewesen, da vor allem die Stabilität gegen Knickung unzureichend war. Gefäßprothesen mit guter Knickstabilität sind vor allem im Bereich von bewegten Gelenken erforderlich.

Nach textiltechnischen Methoden hergestellte, gestrickte oder gewebte Prothesen aus textilen Fäden, z.B. Polyesterfäden, werden seit geraumer Zeit mit gutem Erfolg in der Chirurgie zum vaskulären Ersatz originaler Blutgefäße eingesetzt. Ihre Durchmesser liegen entsprechend den zu reparierenden venösen oder arteriellen Blutgefäßen bei 7 mm und darüber, jedoch lassen sich nach gleichem Verfahren hergestellte kleinlumigere Prothesen mit Durchmessern unter ca. 4 mm wegen der unzureichenden Stabilität, vor allem im Bereich und unter der Wirkung bewegter Gelenke, im allgemeinen nicht mit Erfolg einsetzen. Poröse Gefäßprothesen, zu denen die obenerwähnten textilen Prothesen zu rechnen sind, werden üblicherweise dadurch gegenüber dem in ihnen fließenden Blut abgedichtet, daß man sie vor der Implantation mit Blut durchtränkt, so daß das in den Zwischenräumen geronnene Blut zunächst eine ausreichend dichte Schicht bildet. Die hauptsächlich aus Fibrin bestehende Gerinnungsschicht kann zwischen 0,5 bis 1,5 mm dick sein. Bei kleinen Durchmessern kann eine Schicht in diesen Dicken unter Umständen zu einem baldigen Verschluß führen, so daß der Erfolg des Gefäßersatzes in Frage gestellt ist.

Bei kleinlumigen Prothesen strebt man eine weitgehend glatte, im Querschnitt gleichbleibende Innenfläche an, die der Fibrinbildung und -anlagerung entgegenwirkt. Entsprechend dichte, z.B. aus Polytetrafluorethylen oder aus vliesartigen Materialien aufgebaute Prothesen bedürfen nicht der Abdichtung durch geronnenes Blut, sondern sie können ohne weitere Maßnahmen implantiert werden. Weitgehend dichte Prothesen, die aus verstrecktem Polytetrafluorethylen bestehen, sind z.B. in der DE-B-2 702 513 beschrieben. Die Herstellung einer mikroporösen, vliesartigen Gefäßprothese ist in der DE-A-2 806 030 erläutert. Hiernach werden aus einer Polymerlösung, z.B. einer Lösung eines aus aliphatischen oder aromatischen Isocyanaten und einer Polyolkomponenten aufgebauten Polyurethans in Tetrahydrofuran oder Dimethylformamid, durch einen Spinnvorgang Fasern und daraus die Prothese hergestellt, die durch ihre gerichtete Faseranordnung in Länge und Breite elastisch ist und auch in der Zugfestigkeit einem natürlichen Blutgefäß entspricht bzw. es übertrifft. Der textiltechnische Prothesenaufbau gewährleistet jedoch nicht, daß bei den erforderlichen Wanddicken von ca. 0,5 mm die Knickstabilität in dem für die praktische Verwendung notwendigen Maße gegeben ist; für Prothesen, die über oder unter Gelenken, z.B. Knie- oder Ellenbogengelenk, geführt werden sollen, sind sie deshalb noch nicht geeignet.

Flexible, röhrenförmige Gebilde aus Textilflechtungen oder aus massiven Kunststoffen knicken oft schon bei kleinen Biegungen ab, wobei sich der Durchlaßquerschnitt stark verringert. Außerdem ergeben sich durch das Abknicken manchmal irreversible Materialverformungen, die zu einer bleibenden Wandschädigung führen. Um dieses unerwünschte Verhalten mit einfachen Mitteln zu korrigieren, ist es bekannt, einen flexiblen Schlauch mit einem wendelförmig geführten steifen, elastischen Verstärkungsfaden zu umwickeln und diesen Verstärkungsfaden auf der Schlauchoberfläche thermisch anzuschweißen. Anstelle einer kontinuierlichen wendelförmigen Abstützung können auch mehrere Verstärkungsfäden in Form kreisförmiger Ringe mit gegenseitigen Abständen zur Stabilisierung des Schlauches vorgesehen sein.

Bei kleinlumigen Gefäßprothesen, die nur geringe Wanddicken von ca. 0,5 mm haben, bewirkt der umhüllende Verstärkungsfaden durch seine Eigenspannung eine Deformierung der zunächst glatten Prothesenoberflächen und die Entstehung rillenartiger Eindrücke sowohl auf der Außen- als auch Innenseite. Auf der Außenseite sind derartige Strukturen, da sie im späteren Verlauf der Einwachsung in das Gewebe integriert werden, ohne Bedeutung. Kritischer und deshalb unerwünscht sind wellen- und rillenförmige Oberflächen auf der Protheseninnenseite, weil beim Durchfließen des Blutes eine optimale lineare Strömung durch Turbulenzen beeinträchtigt wird und letztlich zur vermehrten Thrombozytenablagerung, Thrombenbildung und zum Gefäßverschluß führt.

Bei einem bekannten Verfahren zur Herstellung eines Implantats, z.B. einer Gefäßprothese (DE-A-1 566 319), wird ein zylindrischer Schlauch aus einem weitmaschinen Fasermaterial auf einen Dorn gezogen und schraubenförmig mit mehreren Verstärkungsfäden umwickelt. Anschließend erfolgt unter Wärmebehandlung eine Verschmelzung der Verstärkungsfäden mit dem Schlauch, wobei die Verschmelzungsbereiche sich bis zur Innenseite des Schlauchs erstrecken. Hierbei besteht die Gefahr, daß die Innenseite des Schlauches eine Wellenstruktur erhält.

Ferner ist eine Gefäßprothese mit einem faltenbalgartigen Schlauch bekannt (DE-A-2 152 142). In die schraubenförmig verlaufenden Schlauchfalten ist ein Verstärkungsfaden abnehmbar eingewickelt. Bei dieser Gefäßprothese ist die Schlauchinnenseite stets wellenförmig.

Ein Verfahren, von dem der Oberbegriff des Patentanspruchs 1 ausgeht, ist aus GB-A-1 511 030 bekannt. Das bekannte Verfahren dient zur Herstellung eines nur vorübergehend benutzbaren Gefäßeinsatzes, der leicht wieder entfernt werden kann. Dabei wird auf einen Kern ein filmbildendes Bindemittel aufgetragen und auf das Bindemittel wird in noch weichem Zustand ein Faden spiralförmig aufgewickelt, der dabei mindestens teilweise in das Bindemittel eingebettet wird. Anschließend wird die Bindemittelschicht in Anwesenheit des Kernes thermisch behandelt, um Faden und Bindemittel zu einer Einheit zusammenzubacken. Durch das Einblasen von Luft wird der Gefäßeinsatz aufgeweitet, um vom Kern abgenommen zu werden. Bei dem bekannten Verfahren verursacht das Abnehmen des fertigen Gefäßeinsatzes von dem Kern bzw. Dorn erhebliche Schwierigkeiten, mit der Gefahr, daß der Gefäßeinsatz beschädigt wird.

Der Erfindung liegt die Aufgabe zugrunde, ein Verfahren der im Oberbegriff des Patentanspruchs 1 angegebenen Art zu schaffen, mit dem eine knickstabile Gefäßprothese ohne strukturelle Verformung der Schlauchinnenseite auf einfache Weise hergestellt werden kann.

Die Lösung dieser Aufgabe erfolgt erfindungsgemäß mit den im kennzeichnenden Teil des Patentanspruchs 1 angegebenen Merkmalen.

Nach dem erfindungsgemäßen Verfahren wird eine praktisch spannungsfreie und daher keine Oberflächendeformation bewirkende, mit der Oberfläche fest verbundene, stützende Wicklung auf der Schlauchoberfläche aufgebracht. Der auf einem stabförmigen Träger befindliche Prothesenabschnitt mit einer Wanddicke von vorzugsweise 0,5 bis 0,6 mm wird in drehende Bewegung versetzt und dabei der Verstärkungsfaden, der vorzugsweise aus einem Material mit den mechanischen Eigenschaften des Schlauchmaterials besteht, lose auf den Schlauch aufgewickelt. Zum Beispiel ist bei einem Polyurethanschlauch ein Polyurethanfaden aus einem thermoplastischen Polyurethan mit einer Shore-Härte A 70 bis 90 von 1 mm Durchmesser und einer Belastbarkeit von ca. 20 N für diesen Zweck brauchbar. Zur kraftübertragenden Verbindung von Polyurethandraht und Prothesenoberfläche wird die Eigenschaft der verwendeten Polyurethane genützt, daß sie sich unter der Einwirkung bestimmter Lösemittel, wie Tetrahydrofuran oder Dimethylformamid, an- bzw. auflösen und damit miteinander verkleben lassen. Der Polyurethandraht wird vor dem Kontakt mit der Prothesenoberfläche mit dem Lösemittel benetzt, was im allgemeinen zu der gewünschten Verklebung ausreichend ist. Im Rahmen der Erfindung ist es auch möglich, den Verstärkungsfaden an den Schlauch thermisch anzuschmelzen oder anzuschweißen, wobei die Schmelzbereiche aber im Abstand von der Schlauchinnenseite enden müssen.

Wie oben dargelegt, ist es zur Vermeidung einer Prothesenverformung notwendig, die stützende Drahtspirale möglichst spannungsfrei und vor allem ohne Druck auf der Prothesenoberfläche anzubringen, jedoch ist es auch bei weitgehender Verminderung der Drahtspannung beim Wickelvorgang nicht möglich, ohne jeglichen Anpreßdruck eine Verklebung zu erreichen. Berücksichtigt man noch, daß ein Draht mit kreisrundem Querschnitt lediglich eine kleine Kontaktfläche mit dem Schlauch hat und daher bei gegebener Spannung ein hoher spezifischer Flächenanpreßdruck entsteht, kann diese Vorgehensweise nicht zum erwünschten Ziel führen. Um diese Auswirkung auf den Schlauch zu vermeiden, wird nun weiterhin vorgeschlagen, zusammen mit dem die Wicklung bildenden und zu verklebenden Verstärkungsfaden gleichzeitig und parallel laufend ein den Abstand der Drahtwicklungen voneinander, d.h. die Gangzwischenräume, bestimmendes, nichtverklebendes Band aufzuwickeln. Dieses Band bewirkt, daß der Verstärkungsfaden durch seitliche Führung und Abstützung mit geringem Druck auf die Schlauchoberfläche gewickelt werden kann, ohne daß seine Windungen sich verschieben. Durch das mitgeführte Band wird außerdem die Prothesenoberfläche selbst stabilisiert und eine gleichmäßige Kräfteverteilung erreicht. Das Band besteht vorzugsweise aus einem Material, das gegenüber dem für die Polyurethandraht-Prothesenoberflächenverklebung verwendeten Lösemittel inert ist. Besonders eignen sich hierzu polymerisierte Fluorkohlenwasserstoffe, wie Polytetrafluorethylen und Polyethylen, Polypropylen.

Nach dem Aufbringen der Parallelwicklung wird das Lösemittel bei 40 bis 50 °C entfernt und das hilfsweise mitgewickelte Band abgezogen, während der inzwischen fest verklebte Verstärkungsfaden zurückbleibt und mit der Schlauchoberfläche eine Einheit bildet.

Im folgenden wird unter Bezugnahme auf die Zeichnungen ein bevorzugtes Ausführungsbeispiel der Erfindung näher erläutert.

Es zeigen:

Fig. 1 eine perspektivische Darstellung des Aufwickelns des Verstärkungsfadens zusammen mit dem bandförmigen Abstandhalter auf den Schlauch,

Fig. 2 das Abwickeln des Abstandhalters von der Gefäßprothese,

Fig. 3 einen Längsschnitt durch Fig. 1,

Fig. 4 einen Längsschnitt durch die Gefäßprothese, jedoch ohne den Dorn,

Fig. 5 eine vergrößerte Darstellung der Einzelheit V Fig. 4 und

Fig. 6 eine vergrößerte Darstellung des Querschnitts der Verklebungsstelle zwischen Verstärkungsfaden und Schlauch, wie sie sich bei Betrachtung im Rasterelektronenmikroskop darstellt.

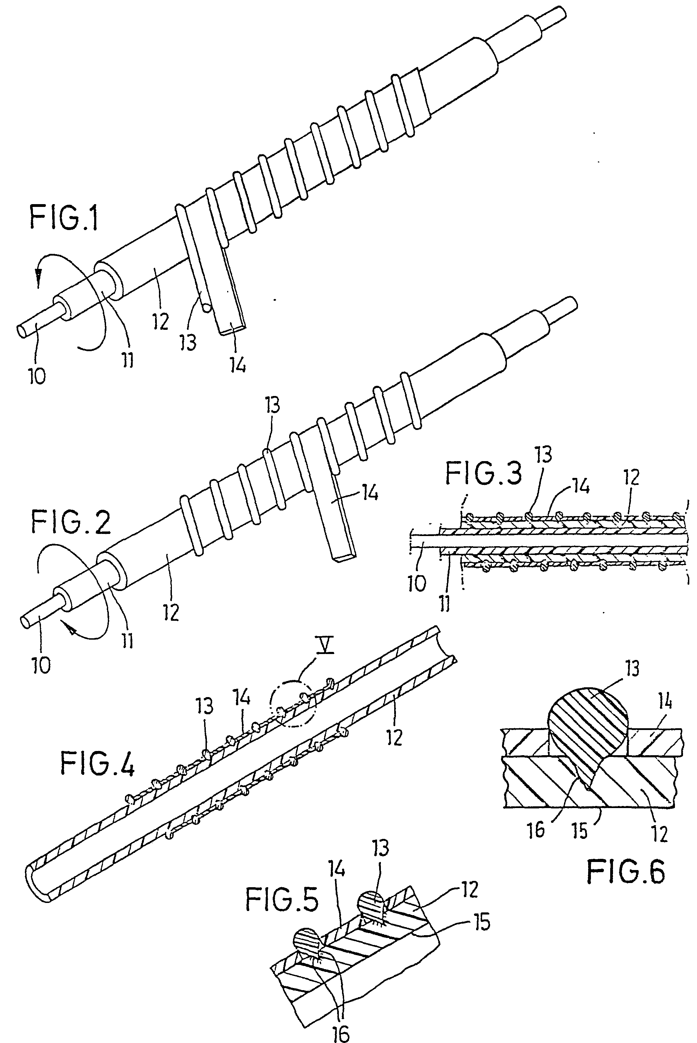

Gemäß Fig. 1 ist ein drehbar gelagerter Dorn 10 aus einem metallischen Stab vorgesehen, der einen Kunststoffmantel 11 aus Polyethylen trägt. Der Kunststoffmantel 11 ist von einem Schlauch 12 umgeben, der nach dem Verfahren der DE-A-2 806 030 durch Aufwickeln zahlreicher Fäden aus Polyurethan hergestellt ist. Fig. 1 zeigt das Verfahren im Anschluß an die Vernetzung des Schlauches 12. Während der Schlauch 12 auf dem Kunststoffmantel 11, auf dem er hergestellt worden ist, verbleibt, werden auf den Schlauch 12 ein Verstärkungsfaden 13 und ein bandförmiger Abstandhalter 14 nebeneinanderliegend aufgewickelt, so daß eine schraubenförmige Wendel entsteht, bei der die einzelnen Windungen des Verstärkungsfadens 13 durch jeweils eine Windung des bandförmigen Abstandhalters 14 voneinander getrennt sind. Bevor der Verstärkungsfaden 13 auf den Schlauch 12 aufläuft, wird seine Unterseite mit einem Lösemittel bestrichen. Der Verstärkungsfaden 13 besteht aus demselben Material wie der Schlauch 12, nämlich aus Polyurethan. Der Abstandhalter 14 besteht aus einem anderen Material, z.B. aus Polyethylen, das gegen das verwendete Lösemittel inert ist. Das Aufwickeln des Verstärkungsfadens 13 erfolgt mit extrem geringer Zugspannung, so daß der Verstärkungsfaden 13 sich nur lose auf die Oberfläche des Schlauches 12 auflegt und dort keine wesentliche Eindrückung erzeugt. Der Verstärkungsfaden 13 hat kreisrunden Querschnitt. Er hat mit seiner Unterseite eine nahezu linienförmige Kontaktfläche mit dem Schlauch. Durch das Lösungsmittel werden die Unterseite des Verstärkungsfadens 13 und der Kontaktbereich des Schlauches 12 mit dem Verstärkungsfaden 13 angelöst, so daß der Verstärkungsfaden 13 sich mit dem Schlauch 12 verbindet, ohne daß die einzelnen Windungen wesentliche Impressionen in der Schlauchoberfläche erzeugen. Während des Wickelvorgangs sorgt der Abstandhalter 14 dafür, daß die nur lose aufgebrachten Windungen des Verstärkungsfadens 13 auf dem Schlauch 12 nicht verrutschen.

Wenn sich der Verstärkungsfaden 13 mit dem Schlauch 12 verbunden hat, wird das Lösungsmittel, z.B. durch Verdampfen, entfernt. Anschließend wird der Dorn 10 in Gegenrichtung zum Aufwickelvorgang gedreht, wobei der bandförmige Abstandhalter 14 von dem Schlauch 12 abgewickelt wird (Fig. 2). Die Gefäßprothese, die nun fertiggestellt ist, kann von dem Dorn 10 abgezogen werden, indem der Kunststoffmantel 11 an seinen Enden auseinandergezogen wird, wodurch sich infolge der Querkontraktion des Kunststoffmantels sein Außendurchmesser verringert, so daß der Schlauch 12 axial von dem Dorn 10 entfernt werden kann.

Die fertige Gefäßprothese aus dem weichelastischen Polyurethan hat eine glatte Innenseite 15, die keine von dem Verstärkungsfaden 13 hervorgerufenen wellenförmigen Impressionen aufweist, so daß die Gefahr, daß in der Gefäßprothese Turbulenzen der Blutströmung hervorgerufen werden, verringert ist. Dennoch ist der Verstärkungsfaden 13 mit seiner Unterseite an den Klebestellen 16, die die keilförmigen Spalte unterhalb des Verstärkungsfadens 13 teilweise ausfüllen, fest mit dem Schlauch 12 verbunden. Wie aus den Fign. 3 bis 6 erkennbar ist, ruhen die Windungen des Verstärkungsfadens 13 seitlich auf den äußeren Kanten des Abstandhalters 14, so daß der Kontaktbereich zwischen Verstärkungsfaden 13 und Schlauch 12 verringert wird und der Verstärkungsfaden nur in ganz lose Berührung mit dem Schlauch kommt. Wie Fig. 6 zeigt, dringt das Lösungsmittel sowohl in den unteren Bereich des Verstärkungsfadens 13 als auch in den äußeren Bereich des Schlauchs 12 ein. In Fig. 6 erkennt man die Folgen einer gewissen Anlösung und Verdichtung des Prothesenmaterials, an der die Wicklung angeklebt wird, die sich jedoch nicht bis zur Innenseite 15 erstreckt. Selbst bei Verwendung eines Verstärkungsfadens 13 mit rundem Querschnitt und mit schmaler Auflagefläche werden Impressionen, die sich bis zur Innenseite 15 erstrecken, vermieden. Es ist jedoch auch möglich, einen Verstärkungsfaden 13 zu verwenden, der nicht-kreisförmig ist und eine größere Auflagefläche hat.

Es ist nicht unbedingt erforderlich, das ein einziger Verstärkungsfaden 13 benutzt wird, der wendelförmig aufgewickelt ist. Vielmehr kann auch eine mehrgängige Wicklung aus mehreren Verstärkungsfäden und mehreren bandförmigen Abstandhaltern vorgesehen sein.

## Patentansprüche

1. Verfahren zur Herstellung einer Gefäßprothese in Form eines mit mindestens einem Verstärkungsfaden (13) umwickelten zylindrischen Schlauches (12) mit glatter Innenseite, bei dem der Schlauch (12) auf einem Dorn (10) erzeugt und anschließend der Verstärkungsfaden (13) auf den auf dem Dorn (10) sitzenden Schlauch (12) mit so geringer Zugspannung aufgewickelt wird, daß sich im Schlauchinnern keine wellenförmigen Deformierungen ergeben, und anschließend mit dem Schlauch verbunden wird, dadurch gekennzeichnet, daß auf den Dorn (10) ein elastischer Kunststoffmantel (11) aufgezogen wird, auf dem der Schlauch (12) erzeugt wird, daß beim Aufwickeln zwischen die Fadenwindungen ein bandförmiger Abstandhalter (14) mit aufgewickelt wird, der nach dem Verbinden des Verstärkungsfadens mit dem Schlauch wieder entfernt wird, und daß der Kunststoffmantel (11) zum Abziehen des Schlauches (12) axial gestreckt wird, wodurch sich sein Außendurchmesser verringert.

2. Verfahren nach Anspruch 1, dadurch gekennzeichnet, daß der Schlauch aus zahlreichen Fasern aufgebaut wird, die anschließend vernetzt werden.

3. Verfahren nach Anspruch 1 oder 2, dadurch gekennzeichnet, daß der Verstärkungsfaden (13) mit dem Schlauch (12) durch Ankleben oder unter Einsatz eines Lösungsmittels kalt verbunden wird, und daß der Abstandhalter (14) aus einem gegen den Klebstoff bzw. das Lösungsmittel inerten Material besteht.

4. Verfahren nach Anspruch 3, dadurch gekennzeichnet, daß der Kleber oder das Lösungsmittel auf den Verstärkungsfaden (13) aufgebracht wird.

## Claims

1. Method for manufacturing a vessel prothesis formed as a cylindrical hose (12) having at least one strengthening thread (13) wound thereabout and being provided with a smooth inner side, wherein the hose (12) is produced on a mandrel (10), and the strengthening thread (13) is subsequently wound onto the hose (12) sitting on the mandrel (10) at such a low tensile strength that no wavelike deformations occur within the hose, and is fixed to the hose afterwards, characterized in that an elastic plastics coating (11) is drawn onto the mandrel (10), on which plastics coating (11) the hose (12) is produced, that during the winding process also a band-shaped spacer (14) is wound between the thread windings and is removed again after said fixing of the strengthening thread to the hose, and that during withdrawal of the hose (12) the plastics coating (11) is axially stretched with a resultant decrease of the outer diameter of the plastics coating (11).

2. Method according to claim 1, characterized in that the hose is built up of numerous fibres which are subsequently cross-linked.

3. Method according to claim 1 or 2, characterized in that the strengthening thread (13) is cold-connected to the hose (12) by adhesion or use of a solvent, and that the spacer (14) is made of a material which is inert against the adhesive and the solvent, respectively.

4. Method according to claim 3, characterized in that the adhesive or the solvent is applied to the strengthening thread (13).

**Revendications**

1. Procédé de fabrication d'une prothèse vasculaire sous forme d'un tube souple cylindrique (12) possédant une face interne lisse, entouré d'au moins un fil de renfort (13), dans lequel le tube souple (12) est produit sur un mandrin (10), le fil de renfort (13) est ensuite enroulé autour du tube souple (12) placé sur le mandrin (10) avec une tension de traction tellement faible qu'il ne se produit pas de déformations ondulées à l'intérieur du tube souple et établit ensuite une liaison avec le tube souple, caractérisé en ce qu'une gaine élastique en matière synthétique (11) est emmanchée sur le mandrin (10) sur lequel est produit le tube souple (12), qu'entre les spires du fil est simultanément enroulé, au cours de l'enroulement, un écarteur (14) en forme de ruban qui est à nouveau retiré après la liaison du fil de renfort avec le tube souple et que, pour dégager le tube souple (12), la gaine en matière synthétique (11) est étirée dans le sens axial, à la suite de quoi son diamètre extérieur diminue.

2. Procédé selon la revendication 1, caractérisé en ce que le tube souple est constitué par de nombreuses fibres qui sont ultérieurement réticulées.

3. Procédé selon la revendication 1 ou 2, caractérisé en ce que le fil de renfort (13) est lié à froid au tube souple (12) par collage ou par emploi d'un solvant et que l'écarteur (14) est constitué par une matière inerte vis-à-vis de l'adhésif, respectivement du solvant.

4. Procédé selon la revendication 3, caractérisé en ce que l'adhésif ou le solvant est appliqué sur le fil de renfort (13).

FIG.1

FIG.2

FIG.3

FIG.4

FIG.5

FIG.6